# EUROPEAN PATENT SPECIFICATION

(11) **EP 2 522 652 B1**
(45) Date of publication and mention of the grant of the patent: **08.11.2017**
(21) Application number: 11744725.0
(22) Date of filing: 18.02.2011
(51) Int. Cl.: C07C 67/14, C07C 67/54, C07C 67/62, C07C 69/63

(54) **METHOD FOR PRODUCING DIFLUOROACETIC ACID ESTER**
VERFAHREN ZUR HERSTELLUNG VON DIFLUORESSIGSÄUREESTER
PROCÉDÉ DE PRODUCTION D'UN ESTER D'ACIDE DIFLUOROACÉTIQUE

(30) Priority: 22.02.2010 JP 2010036419
(43) Date of publication of application: 14.11.2012
(73) Proprietor: Central Glass Company, Limited, Yamaguchi 755-0001 (JP)
(72) Inventor: OKAMOTO, Masamune, Saitama 350-1151 (JP); TAKADA, Naoto, Saitama 350-1151 (JP); IMURA, Hideaki, Saitama 350-1151 (JP)
(74) Representative: Manitz Finsterwald Patentanwälte PartmbB
(86) International application number: PCT/JP2011/053442
(87) International publication number: WO 2011/102439

(56) References cited:
- EP-A1- 0 694 523
- JP-A- 5 504 135
- JP-A- 8 020 560
- JP-A- 8 092 162
- JP-A- 8 127 560
- JP-A- 11 092 423
- JP-A- 2001 048 832
- JP-A- 2003 040 835
- JP-A- 2004 323 408
- JP-A- 2006 028 172
- JP-A- 2006 137 689
- JP-A- 2008 247 815
- JP-A- 2010 116 333
- JP-A- 2010 138 164
- JP-A- 2010 195 753

## Description

### Technical Field

The present invention relates to a method for producing a difluoroacetic acid ester, which can be used as an intermediate for pharmaceutical and agrichemical products or a reagent, and more particularly, to a method for efficiently removing hydrogen fluoride (HF) generated as a by-product of reaction of difluoroacetic acid fluoride with an alcohol.

### Background Art

As production methods of difluoroacetic acid esters, there have been proposed: (1) a process of reacting difluoroacetic acid with an alcohol; (2) a process of reacting a 1-alkoxy-1,1,2,2-tetrafluoroethane with sulfuric acid and silica (Non-Patent Document 1); and (3) a process of bubbling a crude reaction gas containing difluoroacetic acid fluoride into a mixture of ethanol and triethylamine, washing the resulting reaction product with water, and then, extracting ethyl difluoroacetate with methylene chloride from the washed reaction product (Patent Document 1).

The esterification reaction of carboxylic acid fluoride with an alcohol gives hydrogen fluoride as a by-product so that it is absolutely necessary to separate the target carboxylic acid ester from the hydrogen fluoride. In addition, it is desirable to keep the hydrogen fluoride inactive throughout the reaction because the hydrogen fluoride shows significant corrosivity to metal or glass in the coexistence of the raw alcohol. For such inactivation purposes, there have been proposed: a technique of adding sodium fluoride as an acid acceptor (Patent Document 2); and a technique of adding a tertiary amine such as triethylamine (Patent Document 1) or a basic compound (Patent Document 3) into the reaction system so as to fix the hydrogen fluoride as a salt. In these techniques, the resulting inactive hydrogen fluoride salt is removed or rendered harmless by washing with an aqueous solution. However, the recovery rate of the fluorine-containing carboxylic acid ester significantly deteriorates as the fluorine-containing carboxylic acid ester has high hydrolyzability and thereby not only gets decomposed upon the addition of water but also causes increase in water solubility due to the generation of a fluorine-containing carboxylic acid and an alcohol as decomposition products in the aqueous washing. EP 0 694 523 A1 discloses inter alia a method for preparing a difluoroacetic acid ester, which method comprises reacting difluoroacetic acid fluoride with an alcohol, wherein optionally a tertiary amine is present.

### Prior Art Documents

### Patent Documents

Patent Document 1: Japanese Laid-Open Patent Publication No. H08-092162
Patent Document 2: Japanese Laid-Open Patent Publication No. 2006-137689
Patent Document 3: Japanese Laid-Open Patent Publication No. 2008-247815

### Non-Patent Documents

Non-Patent Document 1: J. Am. Chem. Soc., 1950, 72, 1860

### Summary of the Invention

### Problems to be Solved by the Invention

In the synthesis of the ester by reaction of the carboxylic acid fluoride with the alcohol, there cannot be adopted a simple process such as phase separation due to the reason that the salt in which hydrogen fluoride generated as the by-product is fixed by the tertiary amine or basic compound is dissolved in the ester product. On this account, Patent Document 1 discloses that a target difluoroacetic acid ester can be obtained with a yield of 90.3 to 91.9% by washing a reaction liquid containing difluoroacetic acid fluoride, hydrogen fluoride, ethanol and tertiary amine with water to thereby separate the reaction liquid into an organic phase and an aqueous phase, extracting the aqueous layer with methylene chloride, combining the thus-separated organic phase with the previously-separated organic phase, and then, distilling the combined organic phase. Further, Patent Document 3 discloses that an ester-modified pentaerythritol can be obtained by performing esterification of dipentaerythritol and αF-acrylic acid fluoride in the presence of dimethylformaide, adding the resulting reaction liquid into MIBK (methyl isobutyl ketone), washing the mixed liquid with an aqueous solution of hydrogen chloride (HCl) or the like, and subjecting the washed liquid to dehydration and condensation.

As seen in the above examples, it is common to remove the hydrogen fluoride by-product by aqueous washing. This however raises the need to adopt a complicated process with extraction operation in order to reduce the loss of the product caused by dissolution of the product into the aqueous layer.

It is therefore an object of the present invention to provide a method for producing a difluoroacetic acid ester without causing such a drawback that the yield of the product deteriorates due to the inherent generation of hydrogen fluoride as a by-product in the reaction mechanism.

### Means for Solving the Problems

The present inventors have made extensive researches on the efficient recovery of a difluoroacetic acid ester from a reaction liquid for the purpose of improvement in product yield and have found that it is possible to efficiently recover a difluoroacetic acid ester by distillation from the reaction liquid, while allowing hydrogen fluoride to remain in the reaction liquid, by the presence of a specific hydrogen fluoride trapping agent capable of fixing hydrogen fluoride in salt form in the reaction system. The present invention is based on this finding. Herein, the specific hydrogen fluoride trapping agent refers to a specific amide compound or sulfone compound having two kinds of hetero atoms in its molecule. In the case of removing the hydrogen fluoride from the distillation bottom liquid containing the hydrogen fluoride and the hydrogen fluoride trapping agent by the use of water or an aqueous basic solution, there are seen problems such as low recovery rate and waste increase due to poor separability of the hydrogen fluoride trapping agent from the water or aqueous basic solution. The present inventors have however found that it is possible, by further distilling the distillation bottom liquid upon the addition of a tertiary amine, to fix the hydrogen fluoride by the tertiary amine and recover the thus-dissociated hydrogen fluoride trapping agent by distillation. The present inventors have further found that the tertiary amine can be recovered by treating the distillation bottom liquid with an aqueous basic solution and reused for treatment of the reaction product of the present invention.

Namely, the present invention includes the following aspects.

### [Inventive Aspect 1]

A production method of a difluoroacetic acid ester, comprising at least:
a reaction step of obtaining a reaction liquid by reaction of an alcohol represented by ROH (where R is a C₁-C₄ alkyl group) with difluoroacetic acid fluoride (CHF₂COF) in the presence of an amide compound of the following formula (1) or a sulfone compound of the following formula (2) as a hydrogen fluoride trapping agent 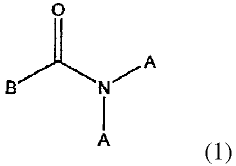 where A can be the same or different and each independently represents a C₁-C₃ alkyl group; and B represents a hydrogen atom or a C₁-C₃ alkyl group; 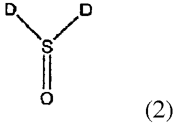 where D can be the same or different and each independently represents a C₁-C₃ alkyl group; and
a distillation step (A) of extracting a difluoroacetic acid ester represented by CHF₂COOR (where R is the same as defined above) by distillation of the reaction liquid obtained in the first step.

### [Inventive Aspect 2]

The production method of the difluoroacetic acid ester according to Inventive Aspect 1, wherein the distillation step (A) is performed to obtain a distillate containing the difluoroacetic acid ester with substantially no hydrogen fluoride and a distillation bottom liquid containing hydrogen fluoride and the hydrogen fluoride trapping agent.

### [Inventive Aspect 3]

The production method of the difluoroacetic acid ester according to Inventive Aspect 1 or 2, wherein the distillation step (A) is preformed under atmospheric pressure conditions or reduced pressure conditions at a distillation bottom temperature of 0°C to 120°C.

### [Inventive Aspect 4]

The production method of the difluoroacetic acid ester according to any one of Inventive Aspects 1 to 3, further comprising: a distillation step (B) of distilling the distillation bottom liquid obtained in the distillation step (A) upon addition of a C₁₂-C₁₅ tertiary amine to the distillation bottom liquid so as to thereby obtain a distillate containing the hydrogen fluoride trapping agent as a main component and a distillation bottom liquid containing the hydrogen fluoride and the tertiary amine.

### [Inventive Aspect 5]

The production method of the difluoroacetic acid ester according to Inventive Aspect 4, further comprising a step of separating and recovering the tertiary amine by contact of an aqueous basic solution with the distillation bottom liquid obtained in the distillation step (B).

### [Inventive Aspect 6]

The production method of the difluoroacetic acid ester according to any one of Inventive Aspects 1 to 5, wherein the distillate obtained in the distillation step (B) and containing the hydrogen fluoride trapping agent as the main component is used as the hydrogen fluoride trapping agent.

### [Inventive Aspect 7]

The production method of the difluoroacetic acid ester according to any one of Inventive Aspects 1 to 6, wherein the hydrogen fluoride trapping agent is either N,N-dimethylacetamide or N,N-dimethylformamide.

### [Inventive Aspect 8]

The production method of the difluoroacetic acid ester according to Inventive Aspect 4 or 5, wherein the tertiary amine is tributylamine.

### [Inventive Aspect 9]

The production method of the difluoroacetic acid ester according to any one of Inventive Aspects 1 to 8, further comprising: a step of obtaining the difluoroacetic acid fluoride as a product of thermal decomposition of a 1-alkoxy-1,1,2,2-tetrafluoroethane represented by CHF₂CF₂OR' (where R' is a monovalent organic group).

In the production method of the difluoroacetic acid ester according to the present invention, the use of the specific amide compound or sulfone compound as the hydrogen fluoride trapping agent provides the effects that: the reaction proceeds with high yield; and the difluoroacetic acid ester can be efficiently recovered from the reaction liquid. It is accordingly possible in the present production method to increase the total yield of the difluoroacetic acid ester. The present production method involves no contact of the difluoroacetic acid ester product with water so that it is possible to avoid problems such as yield deterioration by hydrolysis of the product and entry of impurities into the product. Further, the present production method has small environmental load by easy recovery and reuse of the hydrogen fluoride trapping agent and the tertiary amine.

### Detailed Description of the Embodiments

According to the present invention, there is provided a production method of a difluoroacetic acid ester, including at least: a first step (reaction step) of obtaining a reaction liquid by reaction of an alcohol represented by ROH (where R is a C₁-C₄ alkyl group) and difluoroacetic acid fluoride (CHF₂COF) in the presence of a hydrogen fluoride trapping agent; and a second step (distillation step (A)) of extracting a difluoroacetic acid ester represented by CHF₂COOR (where R is the same as above) by distillation of the reaction liquid obtained in the first step. The present production method may further include: a third step (distillation step (B)) of recovering the hydrogen fluoride trapping agent by further distilling a distillation bottom liquid obtained in the second step upon the addition of a tertiary amine; and a fourth step of recovering the tertiary amine from a distillation bottom liquid obtained in the third step.

As the alcohol represented by ROH, there can be used those having a C₁-C₄ alkyl group as R in the present invention. Examples of R are methyl, ethyl, n-propyl, isopropyl, n-butyl, s-butyl and t-butyl. Specific examples of the alcohol thus include methanol, ethanol, n-propyl alcohol, isopropyl alcohol, n-butyl alcohol, s-butyl alcohol and t-butyl alcohol. Among others, methanol, ethanol, n-propyl alcohol and isopropyl alcohol are preferred. Particularly preferred are ethanol and isopropyl alcohol.

In the present invention, the difluoroacetic acid ester represented by CHF₂COOR has the same alkyl group R as the raw alcohol. Thus, the difluoroacetic acid ester has a C₁-C₄ alkyl group such as methyl, ethyl, n-propyl, isopropyl, n-butyl, s-butyl or t-butyl as R. Specific examples of the difluoroacetic acid ester produced from the above corresponding alcohols include CHF₂COOCH₃, CHF₂COOC₂H₅, CHF₂COOCH₂CH₂CH₃, CHF₂COOCH(CH₃)₂, CHF₂COOCH₂CH₂CH₂CH₃, CHF₂COOCH(CH₃)CH₂CH₃ and CHF₂COOC(CH₃)₃.

The difluoroacetic acid fluoride (CHF₂COF) used in the present invention can be prepared by any process or can be provided as a commercially available industrial product or reagent. As the preparation process, there is known a process of preparing difluoroacetic acid fluoride by thermal decomposition of a 1-alkoxy-1,1,2,2-tetrafluoroethane (CHF₂CF₂OR' where R' is a monovalent organic group) in the presence of a metal oxide catalyst (see e.g. Japanese Laid-Open Patent Publication No. H08-020560).

When R' is alkyl, the thermal decomposition gives not only the target difluoroacetic acid fluoride but also by-products such as alkyl fluoride (R'F) and decomposition products of the alkyl fluoride. In the case where ethyl fluoride is by-produced as the alkyl fluoride, for example, there may be generated ethylene and hydrogen fluoride from the ethylene fluoride. The crude product (crude gas) containing such reaction by-products can be used, without purification treatment, as the raw material for production of the difluoroacetic acid ester in the present invention. It is alternatively feasible to use the crude product as the raw material after mainly removing the alkyl fluoride or feasible to purify the difluoroacetic acid fluoride to a high purity degree and use the purified difluoroacetic acid fluoride as the raw material. Further, it is feasible to store the difluoroacetic acid fluoride as different gases of varying purity degrees by cooling or compression into a pressure-resistance vessel. Although the difluoroacetic acid fluoride can be purified by distillation, it is easier to use the crude gas as it is without separation. When the crude gas is used as it is without separation, it is preferable to cool the crude gas to substantially the same temperature as the reaction temperature of the esterification reaction.

The hydrogen fluoride trapping agent is either an amide compound of the following formula (1) or a sulfone compound of the following formula (2) in the present invention. 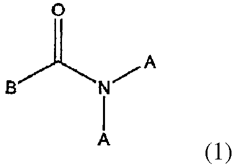 In the formula (1), A can be the same or different and each independently represents a C₁-C₃ alkyl group; and B represents a hydrogen atom or a C₁-C₃ alkyl group. 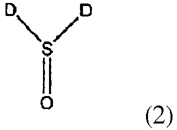 In the formula (2), D can be the same or different and each independently represents a C₁-C₃ alkyl group.

Examples of the amide compound of the formula (1) are N,N-dimethylformamide (DMF), N,N-diethylformamide, N,N-di-n-propylformamide, N,N-diisopropylformamide, N,N-dimethylacetamide (DMAc), N,N-diethylacetamide, N,N-di-n-propylacetamide, N,N-diisopropylacetamide, N,N-dimethylpropionamide, N,N-diethylpropionamide, N,N-di-n-propylpropionamide, N,N-diisopropylpropionamide, N,N-dimethylisobutylamide, N,N-diethylisobutylamide, N,N-di-n-propylisobutylamide, N,N-diisopropylisobutylamide, N,N-dimethylbutylamide, N,N-diethylbutylamide, N,N-di-n-propylbutylamide and N,N-diisopropylbutylamide.

Examples of the sulfone compound of the formula (2) are dimethyl sulfoxide (DMSO), diethyl sulfoxide, diisopropyl sulfoxide and di-n-propyl sulfoxide.

Among the above compounds, preferred are those having a boiling point of 130°C or higher, more preferably 150°C or higher, in view of the fact that the larger the difference between the difluoroacetic acid ester and the amide or sulfone compound, the easier the distillation separation operation. Further, the amide or sulfone compound preferably has a molecular weight of 100 or less because the amount of the amide or sulfone compound used per unit mass of the hydrogen fluoride increases with the molecular weight of the amide or sulfone compound. More specifically, N,N-dimethylformamide, N,N-dimethylacetamide and dimethyl sulfoxide are in particular preferred.

It is feasible to use a reaction vessel of a corrosion-resistant material such as stainless steel, Monel (trade name), Inconel (trade name) or silver, a fluorine resin such as polytetrafluoroethylene or polyperfluoroalkylether, a composite material thereof, or a clad material thereof although the reaction vessel can be of glass material.

In the first step, the alcohol and the hydrogen fluoride trapping agent are first placed in the reaction vessel. The temperature of the reaction vessel is then controlled to a given temperature. After that, the difluoroacetic acid fluoride is introduced in gas form or liquid form into the reaction vessel so as to thereby react the difluoroacetic acid fluoride with the alcohol. There is no particular limitation on the order of placing the alcohol and the hydrogen fluoride trapping agent in the reaction vessel. As the exothermic reaction occurs upon introduction of the difluoroacetic acid fluoride, it is preferable to gradually introduce the difluoroacetic acid fluoride in such a manner that the reaction temperature does not exceed a given degree while monitoring the progress of the reaction. The difluoroacetic acid fluoride may preferably be introduced while stirring. Further, the difluoroacetic acid fluoride may preferably be introduced under cooling. It is feasible to determine the completion of the reaction as a point of disappearance of the alcohol or maximum generation of the difluoroacetic acid ester by analyzing the composition of the reaction liquid by gas chromatography etc. Alternatively, the completion of the reaction can be determined based on the amount of introduction of the difluoroacetic acid fluoride due to the fact that the reaction proceeds nearly quantitatively.

In the present invention, it suffices to use the difluoroacetic acid fluoride in a stoichiometric amount of 1 mol per 1 mol of the alcohol. The amount of the difluoroacetic acid fluoride used is generally 0.5 to 5 mol per 1 mol of the alcohol. The difluoroacetic acid fluoride is preferably used in a slightly excessive amount. More specifically, it is preferable to use 1 to 1.2 mol, more preferably 1 to 1.05 mol, of the difluoroacetic acid fluoride per 1 mol of the alcohol. If the difluoroacetic acid fluoride is used in an excessive amount exceeding 5 mol, the excessive difluoroacetic acid fluoride is recovered as an initial distillation fraction in the second step. The load of the second step is however unfavorably increased. If the difluoroacetic acid fluoride is used in an amount of less than 1 mol, there may occur an azeotropic (pseudo-azeotropic) mixture of the unreacted alcohol and the target difluoroacetic acid ester during the second step. It is desirable to avoid such a small amount of use of the difluoroacetic acid fluoride as the distillation load increases due to the existence of the azeotropic (pseudo-azeotropic) mixture. There may also occur an unfavorable problem that, in the case of using a distillation column system of low plate number, the unreacted alcohol is volatilized as a low-boiling component, with entrainment of the hydrogen fluoride, during the distillation.

It is preferable in the present invention to remove as much water as possible from the alcohol before the use of the alcohol because the water becomes a cause of hydrolysis.

Although it is known that the hydrogen fluoride trapping agent of the present invention, such as N,N-dimethylacetamide or N,N-dimethylformamide, forms a salt with the hydrogen fluoride at a ratio of 1:1, the hydrogen fluoride is not necessarily fixed as such a 1:1 salt in the present invention. The amount of the hydrogen fluoride trapping agent used is generally 0.1 to 10 mol, preferably 0.2 to 5 mol, more preferably 1 to 2 mol, per 1 mol of the alcohol in the present invention. If the hydrogen fluoride trapping agent is used in an amount exceeding 10 mol, there would be no problem in the reaction. However, the amount of treatment per unit capacity of the reaction vessel is unfavorably decreased. If the hydrogen fluoride trapping agent is used in an amount of less than 0.1 mol, the hydrogen fluoride may not be fixed sufficiently so that there unfavorably occurs a fear of corrosion of reaction equipment by the hydrogen fluoride.

There is no particular limitation on the reaction temperature. The reaction temperature generally ranges from -80°C to +50°C, preferably -30°C to +30°C. The lower the temperature, the easier it is to fix the hydrogen fluoride. However, unfavorable results such as increase in cooling cost and solidification of the hydrogen fluoride trapping agent and the difluoroacetic acid ester product may occur if the temperature is lower than -80°C. There would no effect on the reaction even if the temperature exceeds 50°C. It is however unfavorable to set the temperature to be higher than 50°C as the hydrogen fluoride may not be fixed sufficiently at such a high temperature. The reaction pressure is generally 0 to 2 MPa although the reaction pressure varies depending on whether the difluoroacetic acid fluoride is introduced in liquid form or gas form.

In the second step, the reaction liquid obtained in the first step is separated into a fraction (distillate) containing the difluoroacetic acid ester with substantially no hydrogen fluoride and a distillation bottom liquid containing the hydrogen fluoride and the hydrogen fluoride trapping agent. The term "difluoroacetic acid ester with substantially no hydrogen fluoride" refers to difluoroacetic acid ester in which the molar ratio of the hydrogen fluoride to the difluoroacetic acid ester has been made smaller after the distillation than before the distillation. In general, the molar ratio of the hydrogen fluoride to the difluoroacetic acid ester in the fraction is 50/100 or less, preferably 10/100 or less, more preferably 1/100 or less. In the second step, the distillation is carried out to separate the hydrogen fluoride and the hydrogen fluoride trapping agent, i.e., allow the hydrogen fluoride to remain with the hydrogen fluoride trapping agent. It is feasible in the distillation to adopt a simple distillation column or a multi-plate distillation column. As the distillation column, there can be used a plate column such as perforated plate column or bubble plate column or a packed column with a packing material such as Raschig ring, Lessing ring, Dixon packing, pole ring, Saddle ring, MacMahon packing or Sulzer packing.

The second step can be performed, in the same reaction vessel as in the first step (in situ) or in a different vessel, after the completion of the reaction.

In the distillation, the distillation still or bottom temperature can be set higher in order to increase the recovery rate of the difluoroacetic acid ester. On the other hand, it is preferable to set the distillation still or bottom temperature to be low in order to prevent distillation of the hydrogen fluoride. The distillation still or bottom temperature varies depending on the pressure, but generally ranges from 0 to 120°C, preferably 30 to 10°C, more preferably 40 to 90°C. If the distillation still or bottom temperature is lower than 0°C, there occurs an unfavorable increase in energy cost due to the need for high-level vacuum or cooling operation. The difluoroacetic acid ester may unfavorably be decomposed if the distillation still or bottom temperature exceeds 120°C. The distillation is preferably done under reduced pressure in order to set the above distillation temperature range. The distillation pressure varies depending on the distillation still (or bottom) temperature, but generally ranges from 1 Pa to 100 kPa, preferably 10 Pa to 10 kPa, more preferably 100 Pa to 1 kPa. If the distillation pressure is lower than 1 Pa, there occurs an unfavorable increase in energy cost due to the need for high-level vacuum or cooling operation. If the distillation pressure exceeds 100 kPa, the difluoroacetic acid ester may unfavorably be decomposed with increase in distillation still temperature.

When the distillation is low in accuracy, the difluoroacetic acid ester fraction obtained in the second step may contain the hydrogen fluoride, the hydrogen fluoride trapping agent and the unreacted alcohol. In such a case, the distillation can be performed repeatedly. Further, it is feasible to remove the hydrogen fluoride by adsorption onto sodium fluoride pellets or any other solid adsorbent such as allophone.

In the third step, the distillation bottom liquid obtained in the second step and containing the hydrogen fluoride and the hydrogen fluoride trapping agent is further distilled upon the addition of the tertiary amine so as to thereby separate and recover a distillate containing the hydrogen fluoride trapping agent as the main component and a distillation bottom liquid containing the hydrogen fluoride and the tertiary amine.

The tertiary amine is an amine represented by R₃N (where R is each independently an alkyl group). Preferably, the tertiary amine is of 12 to 15 carbon atoms. If the carbon number of the tertiary amine is less than 12, the tertiary amine has a low boiling point and may be mixed into the hydrogen fluoride trapping agent during the distillation. Further, the resulting hydrogen fluoride salt of the tertiary amine has high water solubility so that it becomes difficult to conduct two-phase separation during the washing of the distillation bottom liquid with an aqueous basic solution. If the carbon number of the tertiary amine exceeds 15, the tertiary amine has a large molecular weight so that the apparent amount of the tertiary amine used is unfavorably increased.

Specific examples of the tertiary amine are: symmetric tertiary amines such as tri-n-butylamine, tri-isobutylamine, tri-sec-butylamine, tri-tert-butylamine, tri-n-amylamine, tri-isoamylamine, tri-sec-amylamine and tri-tert-amylamine; and asymmetric tertiary amines such as N,N-dimethyldecylamine, N,N-dimethylundecylamine and N,N-dimethyldocecylamine. Among others, it is preferable to use symmetric amines in view of availability. More preferred are tri-n-butylamine, tri-isobutylamine, tri-n-amylamine, tri-isoamylamine. Tri-n-butylamine is particularly preferred.

The tertiary amine is used in an amount of 0.5 to 2 mol, preferably 0.7 to 1.5 mol, per 1 mol of the hydrogen fluoride contained in the distillation bottom liquid. If the tertiary amine is used in an amount of less than 0.5 mol, the salt of the tertiary amine and the hydrogen fluoride may unfavorably be deposited. The purpose of the distillation can be achieved even if the tertiary amine is used in an amount exceeding 2 mol. There however occurs an unfavorable decrease in the efficiency of removing the hydrogen fluoride, in the subsequent fourth step, from the high-boiling fraction (distillation bottom liquid) containing the tertiary amine.

In the distillate obtained in the third step and containing the hydrogen fluoride trapping agent as the main component, the amount of the hydrogen fluoride is larger than the amount of the other component and is preferably 50 wt% or more. The tertiary amine and the slight amount of the hydrogen fluoride may be contained in the distillate. It is however feasible to use the distillate, as it is without further purification operation, as the hydrogen fluoride trapping agent in the first step. The distillate may alternatively be subjected to precision distillation in order to increase the purity of the hydrogen fluoride trapping agent.

In the fourth step, the distillation bottom liquid obtained in the third step is brought into contact with the aqueous basic solution so as to thereby separate and recover the tertiary amine. The distillation bottom liquid obtained in the third step contains the tertiary amine and the hydrogen fluoride.

As a basic material in the aqueous basic solution, there can be used a hydroxide of an alkali metal or alkaline-earth metal, a metal or an oxide. Among others, the hydroxide is preferred. Specific examples of the basic material are potassium hydroxide, sodium hydroxide, calcium hydroxide, lithium hydroxide, sodium oxide and calcium oxide. In view of ease of handling, potassium hydroxide and sodium hydroxide are particularly preferred.

There is no particular limitation on the concentration of the aqueous basic solution. The concentration of the aqueous basic solution is generally in the range of 0.1 to 50 mass%. If the concentration of the aqueous basic solution is lower than 0.1 mass%, the capacity of treatment equipment is unfavorably increased. If the concentration of the aqueous basic solution exceeds 50 mass%, the operation is unfavorably complicated due to deposition of a solid fluoride.

There are formed an aqueous phase and an organic phase when the distillation bottom liquid is mixed and brought into contact with the aqueous basic solution. It is feasible to obtain the tertiary amine with substantially no hydrogen fluoride by recovering the organic phase. The thus-obtained amine can be dried with a solid drying agent such as synthetic zeolite. It is further feasible to purify the tertiary amine by distillation etc. before or after the drying. The tertiary amine, after the drying, can be used as it is in the third step. In the fourth step, the separation/recovery operation is done under either atmospheric temperature and pressure conditions or different conditions.

### Examples

The present invention will be described in more detail below by way of the following examples. It should be noted that the following examples are not intended to limit the present invention thereto. In the following examples, the contents of organic components such as ethyl difluoroacetate (CHF₂COOEt), ethanol (EtOH), difluoroacetic acid (CHF₂COOH) were measured by FID gas chromatography (GC); and the content of inorganic acids such as hydrogen fluoride were measured by ion chromatography (IC).

### [Synthesis Example 1]

An aluminum phosphate catalyst was prepared by compression molding aluminum phosphate available from Aldrich Chemical Co. into pellets of 5 mm diameter x 5 mm length and firing the pellets at 700°C for 5 hours under a nitrogen flow atmosphere. Then, a large-size gas-phase reaction tube (formed of stainless steel with an inner diameter of 43 mm φ and a length of 1800 mm L) having a carburetor was packed with 2200 cc of the prepared catalyst. While flowing nitrogen at 1000 cc/min through the reaction tube, the reaction tube was heated externally by an electric furnace. After the temperature of the catalyst reached 50°C, hydrogen fluoride (HF) was introduced at a rate of maximum 6 g/min into the reaction tube through the carburetor while monitoring so as not to cause sudden heat generation. The temperature of the catalyst was raised gradually up to 300°C while maintaining the flow of the HF. The introduction rate of the HF was gradually increased to 12 g/min. In this state, the catalyst was held at 300°C for 72 hours. After that, the heater setting temperature was lowered. At the time when the inside temperature of the reaction tube reached 250°C, the flow of the HF was stopped; and the flow rate of the nitrogen was increased to 2000 cc/min. The catalyst was then held for 8 hours. Subsequently, 1-methoxy-1,1,2,2-tetrafluoroethane (HFE-254pc) was introduced at a rate of 40 g/min into the reaction tube through the carburetor. After a lapse of 30 minutes, the flow of the nitrogen was stopped to thereby allow the flow of only the HFE-254pc into the reaction tube. The thermal decomposition gas product was sampled under a steady state (reaction temperature: 210°C) and analyzed by gas chromatography. By the above reaction process, difluoroacetic acid fluoride (CHF₂COF) and methyl fluoride (CH₃F) were obtained with a yield of 99.8%. The above-obtained gas product was purified by distillation and used in Examples 1 and 2.

### [Synthesis Example 2]

An aluminum phosphate catalyst was prepared by compression molding aluminum phosphate available from Aldrich Chemical Co. into pellets of 5 mm diameter x 5 mm length and firing the pellets at 700°C for 5 hours under a nitrogen flow atmosphere. Then, a small-size gas-phase reaction tube (formed of stainless steel with an inner diameter of 37 mm and a length of 500 mm) having a carburetor was packed with 200 cc of the prepared catalyst. While flowing nitrogen at 100 cc/min through the reaction tube, the reaction tube was heated externally by an electric furnace. After the temperature of the catalyst reached 50°C, hydrogen fluoride (HF) was introduced at a rate of maximum 0.6 g/min into the reaction tube through the carburetor while monitoring so as not to cause sudden heat generation. The temperature of the catalyst was raised gradually up to 300°C while maintaining the flow of the HF. The introduction rate of the HF was gradually increased to 1.2 g/min. In this state, the catalyst was held at 300°C for 72 hours. After that, the heater setting temperature was lowered. At the time when the inside temperature of the reaction tube reached 250°C, the flow of the HF was stopped; and the flow rate of the nitrogen was increased to 200 cc/min. The catalyst was then held for 8 hours. Subsequently, 1-methoxy-1,1,2,2-tetrafluoroethane (HFE-254pc) was introduced at a rate of 0.55 g/min into the reaction tube through the carburetor. After a lapse of 30 minutes, the flow of the nitrogen was stopped to thereby allow the flow of only the HFE-254pc into the reaction tube. The thermal decomposition gas product was sampled under a steady state (reaction temperature: 210°C) and analyzed by gas chromatography. By the above reaction process, difluoroacetic acid fluoride (CHF₂COF) and methyl fluoride (CH₃F) were obtained with a yield of 99.9%. The above-obtained gas product was naturally cooled by a stainless spiral tube and used in Example 3 as it is without purification.

### [Example 1]

Into a nitrogen-sealed, three-neck PFA vessel (500 cc) with a dry ice condenser (coldfinger condenser), a blowing tube and a thermometer, 46 g (1 mol) of ethanol and 174 g (2 mol) of N,N-dimethylacetamide (DMAc) were placed. The vessel was cooled in an ice water bath while stirring the content of the vessel. While cooling the vessel in the ice water bath, 104 g (1.06 mol) of the difluoroacetic acid fluoride (CHF₂COF, DFAF) purified by distillation in Synthesis Example 1 was introduced into the vessel through the blowing tube over 240 minutes. There was thus obtained, in the vessel, 329 g of a crude ethyl difluoroacetate mixture (containing 174 g of DMAc) as a reaction liquid. The reaction liquid was sampled. It was confirmed by gas chromatographic analysis of the sample that the reaction liquid contained ethyl difluoroacetate (24.85 area%) and ethanol (0.048 area%). Further, it was confirmed by ion chromatographic analysis of fluorine ion in the sample that the hydrogen fluoride content of the reaction liquid was 7.086 mass%. The reaction liquid was subjected to simple distillation under reduced pressure (9.0 kPa), thereby extracting 108 g of ethyl difluoroacetate (CHF₂COOEt) with a purity of 96.8 area% as a main fraction (column top temperature: 41.0°C). It was confirmed by ion chromatographic analysis that: the fluorine ion concentration of the extracted main fraction was 0.15 mass%; and the fluorine ion concentration of the distillation bottom residue was concentrated to 9.53 mass%. The analysis results of the respective distillation fractions are indicated in TABLE 1.

**TABLE 1**

| Sample | g | GC (area%) | | | | | F ion concentration (mass%) |
|---|---|---|---|---|---|---|---|
| | | EtOH | CHF₂COOEt | CHF₂COOH | DMAc | Others | |
| Soon after reaction | 329 | 0.048 | 24.861 | n.d. | 74.55 | 0.541 | 7.086 |
| Initial fraction | 6 | 0.161 | 95.166 | 0.578 | n.d. | 4.095 | 0.981 |
| Main fraction | 108 | 0.313 | 96.775 | 0.128 | 2.475 | 0.309 | 0.155 |
| Bottom liquid | 203 | 0.046 | 3.219 | n.d. | 95.236 | 1.499 | 9.534 |

| | | | | | | | |
|---|---|---|---|---|---|---|---|
| DMAc): N,N-dimethylacetamide CHF₂COOH: difluoroacetic acid n.d.: not detected | | | | | | | |

### [Example 2]

The same operation as in Example 1 was performed except for using 55 g (1.2 mol) of ethanol, 208 g (2.4 mol) of DMAc and 129 g (1.25 mol) of the difluoroacetic acid fluoride. There was thus obtained 392 g of a crude ethyl difluroacetate mixture (containing 208 g of DMAc) as a reaction liquid. The reaction liquid was distillated under reduced pressure (9.0 kPa) by a distillation column (theoretical plate number: 5 plates) packed with Dixon packing, thereby extracting 144 g of ethyl difluoroacetate (isolated yield: 96%) with a purity of 98.8 area% and a fluorine ion concentration of 0.07 mass% as a main fraction (column top temperature: 41.0°C) as shown in TABLE 1.

**TABLE 2**

| Sample | g | GC (area%) | | | | |
|---|---|---|---|---|---|---|
| | | EtOH | CHF₂COOEt | CHF₂COOH | DMAc | Others |
| Soon after reaction | 392 | 0.011 | 25.684 | n.d. | 72.668 | 1.637 |
| Main fraction | 144 | 0.407 | 98.769 | 0.046 | 0.026 | 0.752 |

| | | | | | | |
|---|---|---|---|---|---|---|
| DMAc: N,N-dimethylacetamide CHF₂COOH: difluoroacetic acid n.d.: not detected | | | | | | |

### [Example 3]

The same experimental operation as in Example 2 was performed except that the thermal decomposition gas of Synthesis Example 2 (crude CHF₂COF gas containing CH₃F) was used, without purification, as CHF₂COF. There was thus yielded 145 g of CHF₂COOEt (isolated yield: 96%) with a purity of 98.8 area% and a fluorine ion concentration of 0.08 mass% as shown in TABLE 3.

**TABLE 3**

| Sample | g | GC (area%) | | | | |
|---|---|---|---|---|---|---|
| | | EtOH | CHF₂COOEt | CHF₂COOH | DMAc | Others |
| Soon after reaction | 394 | 0.009 | 25.142 | 0.012 | 73.217 | 1.620 |
| Main fraction | 145 | 0.307 | 98.753 | 0.043 | 0.022 | 0.875 |

| | | | | | | |
|---|---|---|---|---|---|---|
| DMAc): N,N-dimethylacetamide CHF₂COOH: difluoroacetic acid n.d.: not detected | | | | | | |

### [Example 4]

The same experimental operation as in Example 1 was performed except that N,N-dimethylformamide (DMF, 175 g) was used in place of DMAc (208 g). The resulting reaction liquid was subjected to simple distillation under reduced pressure (9.0 kPa). There was thus yielded 139 g of CHF₂COOEt with a purity of 98.74 area% and a fluorine ion concentration of 0.059 mass% as a main fraction (column top temperature: 41.0°C).

### [Example 5]

The same experimental operation as in Example 1 was performed except that: dimethyl sulfoxide (DMSO, 187 g) was used in place of DMAc (208 g); and methanol (38.4 g) was used in place of ethanol (55 g). The resulting reaction liquid was subjected to simple distillation under reduced pressure (8.1 kPa). There was thus yielded 128 g of methyl difluoroacetate (CHF₂COOMe) with a purity of 97.5 area% and a fluorine ion concentration of 0.109 mass% as a main fraction (column top temperature: 29.0°C).

### [Example 6]

Into a nitrogen-sealed, three-neck PFA vessel (500 ml) with a dry ice condenser (coldfinger condenser), a blowing tube and a thermometer, 194 g (1.05 mol) of tri-n-butylamine was placed. The vessel was cooled in an ice water bath while stirring the content of the vessel. Then, the DMAc solution (78 g) obtained as the distillation bottom residue in Example 1 and containing 7.4 g of hydrogen fluoride was dropped into the vessel at a rate of 5 g/min. There was almost no heat generation seen during the dropping. After the dropping, the dry ice condenser was replaced with a Dimroth condenser. The resulting mixed solution was stirred at 120°C for 90 minutes. After cooling the solution to room temperature, the solution was separated in two phases. There was thus recovered 152 g of n-Bu₃N containing 0.16 g of hydrogen fluoride as the upper phase and 118 g of the lower phase. It was confirmed by ¹H-NMR measurement of the lower phase that the molar ratio of DMAc and n-Bu₃N in the lower phase was 1:0297. The lower phase was subjected to simple distillation under reduced pressure (7.2 kPa), thereby extracting 72 g of DMAc with a purity of 80.0 area% (n-Bu₃N content: 20 area%) as a main fraction (column top temperature: 88 to 90°C). It was confirmed by ion chromatographic analysis that the fluorine ion concentration of the extracted main fraction was 0.038 mass%. Further, 47 g of the distillation bottom residue was washed with 300 g of 5 mass% aqueous NaOH solution and 110 g of 1 mass% aqueous KOH solution. There was thus recovered 16 g of n-Bu₃N as an organic phase. The aqueous solution used for washing the distillation bottom residue was analyzed by ion chromatography. It was confirmed from the analysis results that the fluorine ion concentration of the aqueous solution was 1.8 mass%, that is, 7.3 g out of 7.4 g of the hydrogen fluoride contained in the reaction liquid was recovered. The total recovery amounts of the organic components were as follows: 190 g n-Bu₃N (recovery rate: 98%) and 58 g DMAc.

The same operation as in Example 1 was performed except for using 72 g of the recovered DMAc (containing 20 area% of n-Bu₃N), 24 g of EtOH and 51 g of difluoroacetic acid fluoride (DFAF). There was thus obtained, in the vessel, 135 g of a crude ethyl difluoroacetate mixture as a reaction liquid. The reaction liquid was sampled. It was confirmed by gas chromatographic analysis of the sample that the reaction liquid contained ethyl difluoroacetate (51.88 area%), ethanol (0.012 area%), DMAc (34.40 area%) and n-Bu₃N (11.0 area%) The results were similar to those of Example 1.

As described above, the present invention is useful as the method for production of the difluoroacetic acid ester with less environmental load and without causing yield deterioration and entry of impurities.

## Claims

1. A production method of a difluoroacetic acid ester, comprising at least:
a reaction step of obtaining a reaction liquid by reaction of an alcohol represented by ROH (where R is a C₁-C₄ alkyl group) with difluoroacetic acid fluoride (CHF₂COF) in the presence of an amide compound of the following formula (1) or a sulfone compound of the following formula (2) as a hydrogen fluoride trapping agent 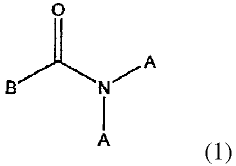 where A can be the same or different and each independently represents a C₁-C₃ alkyl group; and B represents a hydrogen atom or a C₁-C₃ alkyl group; 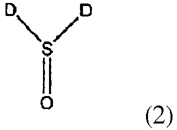 where D can be the same or different and each independently represents a C₁-C₃ alkyl group; and
a distillation step (A) of extracting a difluoroacetic acid ester represented by CHF₂COOR (where R is the same as defined above) by distillation of the reaction liquid obtained in the first step.

2. The production method of the difluoroacetic acid ester according to claim 1, wherein the distillation step (A) is performed to obtain a distillate containing the difluoroacetic acid ester with substantially no hydrogen fluoride and a distillation bottom liquid containing hydrogen fluoride and the hydrogen fluoride trapping agent.

3. The production method of the difluoroacetic acid ester according to claim 1 or 2, wherein the distillation step (A) is preformed under atmospheric pressure conditions or reduced pressure conditions at a distillation bottom temperature of 0°C to 120°C.

4. The production method of the difluoroacetic acid ester according to any one of claims 1 to 3, further comprising:
a distillation step (B) of distilling the distillation bottom liquid obtained in the distillation step (A) upon addition of a C₁₂-C₁₅ tertiary amine to the distillation bottom liquid so as to thereby obtain a distillate containing the hydrogen fluoride trapping agent as a main component and a distillation bottom liquid containing the hydrogen fluoride and the tertiary amine.

5. The production method of the difluoroacetic acid ester according to claim 4, further comprising:
a step of separating and recovering the tertiary amine by contact of an aqueous basic solution with the distillation bottom liquid obtained in the distillation step (B).

6. The production method of the difluoroacetic acid ester according to any one of claims 1 to 5, wherein the distillate obtained in the distillation step (B) and containing the hydrogen fluoride trapping agent as the main component is used as the hydrogen fluoride trapping agent.

7. The production method of the difluoroacetic acid ester according to any one of claims 1 to 6, wherein the hydrogen fluoride trapping agent is either N,N-dimethylacetamide or N,N-dimethylformamide.

8. The production method of the difluoroacetic acid ester according to claim 4 or 5, wherein the tertiary amine is tributylamine.

9. The production method of the difluoroacetic acid ester according to any one of claims 1 to 8, further comprising:
a step of obtaining the difluoroacetic acid fluoride as a product of thermal decomposition of a 1-alkoxy-1,1,2,2-tetrafluoroethane represented by CHF₂CF₂OR' (where R' is a monovalent organic group).

## Patentansprüche

1. Herstellungsverfahren für Difluoressigsäureester, umfassend zumindest:
einen Reaktionsschritt eines Erhaltens einer Reaktionsflüssigkeit durch Reaktion eines durch ROH dargestellten Alkohols (wobei R eine C₁-C₄-Alkylgruppe ist) mit Difluoressigsäurefluorid (CHF₂COF) in Gegenwart einer Amidverbindung der folgenden Formel (1) oder einer Sulfonverbindung der folgenden Formel (2) als Fluorwasserstoff-Einfangmittel 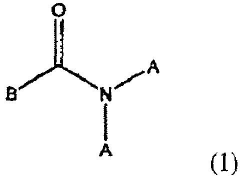 wobei A gleich und verschieden sein können und jeweils unabhängig für eine C₁-C₃-Alkylgruppe stehen; und B für ein Wasserstoffatom oder eine C₁-C₃-Alkylgruppe steht; 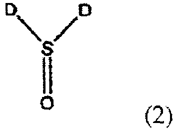 wobei D gleich oder verschieden sein können und jeweils unabhängig für eine C₁-C₃-Alkylgruppe stehen; und
einen Destillationsschritt (A) eines Extrahierens eines durch CHF₂COOR dargestellten Difluoressigsäureesters (wobei R gleich wie oben definiert ist) durch Destillation der in dem ersten Schritt erhaltenen Reaktionsflüssigkeit.

2. Herstellungsverfahren des Difluoressigsäureesters nach Anspruch 1, wobei der Destillationsschritt (A) durchgeführt wird, um ein Destillat, das den Difluoressigsäureester im Wesentlichen ohne Fluorwasserstoff enthält, und eine Destillationssumpfflüssigkeit, die Fluorwasserstoff und das Fluorwasserstoff-Einfangmittel enthält, zu erhalten.

3. Herstellungsverfahren des Difluoressigsäureesters nach Anspruch 1 oder 2, wobei der Destillationsschritt (A) unter Luftdruckbedingungen oder Unterdruckbedingungen bei einer Destillationssumpftemperatur von 0°C bis 120°C durchgeführt wird.

4. Herstellungsverfahren des Difluoressigsäureesters nach einem der Ansprüche 1 bis 3,
ferner umfassend: einen Destillationsschritt (B) eines Destillierens der in dem Destillationsschritt (A) erhaltenen Destillationssumpfflüssigkeit unter Zugabe eines tertiären C₁₂-C₁₅-Amins zu der Destillationssumpfflüssigkeit, um dadurch ein Destillat, das das Fluorwasserstoff-Einfangmittel als Hauptkomponente enthält, und eine Destillationssumpfflüssigkeit, die den Fluorwasserstoff und das tertiäre Amin enthält, zu erhalten.

5. Herstellungsverfahren des Difluoressigsäureesters nach Anspruch 4, ferner umfassend:
einen Schritt eines Trennens und Rückgewinnens des tertiären Amins durch Kontakt einer wässrigen basischen Lösung mit der in dem Destillationsschritt (B) erhaltenen Destillationssumpfflüssigkeit.

6. Herstellungsverfahren des Difluoressigsäureesters nach einem der Ansprüche 1 bis 5,
wobei das in dem Destillationsschritt (B) erhaltene und das Fluorwasserstoff-Einfangmittel als die Hauptkomponente enthaltende Destillat als das Fluorwasserstoff-Einfangmittel verwendet wird.

7. Herstellungsverfahren des Difluoressigsäureesters nach einem der Ansprüche 1 bis 6,
wobei das Fluorwasserstoff-Einfangmittel entweder N,N-Dimethylacetamid oder N,N-Dimethylformamid ist.

8. Herstellungsverfahren des Difluoressigsäureesters nach Anspruch 4 oder 5, wobei das tertiäre Amin Tributylamin ist.

9. Herstellungsverfahren des Difluoressigsäureesters nach einem der Ansprüche 1 bis 8, ferner umfassend:
einen Schritt eines Erhaltens eines Difluoressigsäurefluorids als ein Produkt einer thermischen Zersetzung eines 1-Alkoxy-1,1,2,2-tetafluorethans, das durch CHF₂CF₂OR' dargestellt ist (wobei R' eine monovalente organische Gruppe ist).

## Revendications

1. Procédé de production d'un ester d'acide difluoroacétique, comprenant au moins :
une étape de réaction consistant à obtenir un liquide réactionnel par réaction d'un alcool représenté par ROH (où R est un groupe alkyle en C₁ à C₄) avec un fluorure d'acide difluoroacétique (CHF₂COF) en présence d'un composé amide de formule suivante (1) ou un composé sulfone de formule suivante (2) comme agent de piégeage du fluorure d'hydrogène 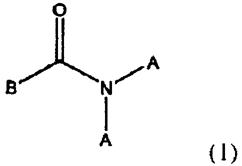 où A peut être identique ou différent et chacun indépendamment représente un groupe alkyle en C₁ à C₃ ; et B représente un atome d'hydrogène ou un groupe alkyle en C₁ à C₃ ; 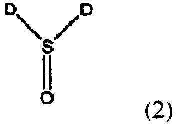 où D peut être identique ou différent et chacun indépendamment représente un groupe alkyle en C₁ à C₃ ; et
une étape de distillation (A) consistant à extraire un ester d'acide difluoroacétique représenté par CHF₂COOR (où R est identique à celui défini ci-dessus) par distillation du liquide réactionnel obtenu au cours de la première étape.

2. Procédé de production d'ester d'acide difluoroacétique selon la revendication 1, dans lequel l'étape de distillation (A) est réalisée afin d'obtenir un distillat contenant l'ester d'acide difluoroacétique n'ayant sensiblement pas de fluorure d'hydrogène et un liquide de queue de colonne de distillation contenant du fluorure d'hydrogène et l'agent de piégeage du fluorure d'hydrogène.

3. Procédé de production d'ester d'acide difluoroacétique selon la revendication 1 ou 2, dans lequel l'étape de distillation (A) est préformée dans des conditions de pression atmosphérique ou des conditions de pression réduite à une température de queue de colonne de distillation de 0°C à 120°C.

4. Procédé de production d'ester d'acide difluoroacétique selon l'une quelconque des revendications 1 à 3, comprenant en outre :
une étape de distillation (B) consistant à distiller le liquide de queue de colonne de distillation obtenu au cours de l'étape de distillation (A) lors de l'addition d'une amine tertiaire en C₁₂ à C₁₅ au liquide de queue de colonne de distillation de manière à obtenir ainsi un distillat contenant l'agent de piégeage du fluorure d'hydrogène comme composant principal et un liquide de queue de colonne de distillation contenant le fluorure d'hydrogène et l'amine tertiaire.

5. Procédé de production d'ester d'acide difluoroacétique selon la revendication 4, comprenant en outre :
une étape de séparation et de récupération de l'amine tertiaire par contact d'une solution basique aqueuse avec le liquide de queue de colonne de distillation obtenu à l'étape de distillation (B).

6. Procédé de production d'ester d'acide difluoroacétique selon l'une quelconque des revendications 1 à 5, dans lequel le distillat obtenu à l'étape de distillation (B) et contenant l'agent de piégeage du fluorure d'hydrogène comme composant principal est utilisé comme agent de piégeage du fluorure d'hydrogène.

7. Procédé de production d'ester d'acide difluoroacétique selon l'une quelconque des revendications 1 à 6, dans lequel l'agent de piégeage du fluorure d'hydrogène est soit le N,N-diméthylacétamide soit le N,N-diméthyl-formamide.

8. Procédé de production d'ester d'acide difluoroacétique selon la revendication 4 ou 5, dans lequel l'amine tertiaire est la tributylamine.

9. Procédé de production d'ester d'acide difluoroacétique selon l'une quelconque des revendications 1 à 8, comprenant en outre :
une étape d'obtention du fluorure d'acide difluoroacétique comme produit de la décomposition thermique d'un 1-alkoxy-1,1,2,2-tétrafluoroéthane représenté par CHF₂CF₂OR' (où R' est un groupe organique monovalent).
